# EUROPEAN PATENT APPLICATION

(11) **EP 4 549 394 A1**
(43) Date of publication of application: **07.05.2025**
(21) Application number: 23831208.6
(22) Date of filing: 20.06.2023
(51) Int. Cl.: C01G 39/00, A01P 1/00, A61L 2/23, C04B 35/495, C04B 35/50, A01P 3/00, A01N 59/06, A01N 59/16

(54) **CE-MO COMPOSITE OXIDE CERAMIC, GREEN COMPACT, SINTERED BODY AND ARTICLE**

(30) Priority: 28.06.2022 JP 2022103322
(71) Applicant: Niterra Co., Ltd., Nagoya-shi, Aichi 461-0005 (JP); Tokyo Institute of Technology, Tokyo 152-8550 (JP)
(72) Inventor: IWATA Masaya, Nagoya-shi, Aichi 461-0005 (JP); OKAYAMA Hiroki, Nagoya-shi, Aichi 461-0005 (JP); NAKAJIMA Akira, Tokyo 152-8550 (JP)
(74) Representative: Grünecker Patent- und Rechtsanwälte PartG mbB
(86) International application number: PCT/JP2023/022749
(87) International publication number: WO 2024/004762

(57) **Abstract**

A Ce-Mo-based composite oxide ceramic contains: cerium; molybdenum; and at least one type of trace element selected from the group consisting of Al, Zr, and Si.

## Description

### TECHNICAL FIELD

The present invention relates to a Ce-Mo-based composite oxide ceramic, a green compact, a sintered body, and an article.

### BACKGROUND ART

Inorganic antibacterial agents and inorganic antiviral agents have advantages such as: the advantage that these agents can be used in a wide range of temperatures; and the advantage that viruses and the like hardly develop resistance thereto. Thus, research on these agents have been actively conducted in recent years. As this type of antibacterial and antiviral agent, agents based on metals such as Ag and Cu, agents based on photocatalysts such as TiO₂, agents based on metal oxides such as ZnO and CaO, and the like have been reported to date and actually used.

As described in Patent Document 1, attention has been particularly paid to a composite oxide ceramic that contains cerium (Ce) and molybdenum (Mo) and that has a high antibacterial activity and a high antiviral activity among these inorganic antibacterial agents and inorganic antiviral agents.

### RELATED ART DOCUMENT

Patent Document 1: International Publication WO 2022/014631

### Problem to be Solved by the Invention

Conventionally, no study has been conducted in relation to, for example, what influence another element other than cerium, molybdenum, and oxygen inflicts on the antibacterial property and the antiviral property of the composite oxide ceramic containing cerium and molybdenum when the other element is mixed with the composite oxide ceramic.

### DISCLOSURE OF THE PRESENT INVENTION

An object of the present invention is to provide a Ce-Mo-based composite oxide ceramic and other products each having an excellent antibacterial property and an excellent antiviral property.

### Means for Solving the Problem

The present inventors conducted thorough studies in order to achieve the object. As a result, the present inventors have found that, when a Ce-Mo-based composite oxide ceramic containing a composite oxide of cerium (Ce) and molybdenum (Mo) contains at least one type of trace element selected from the group consisting of Al, Zr, and Si, metal ions become easy to elute so that an excellent antibacterial property and an excellent antiviral property can be exhibited. Consequently, the present inventors have completed the present invention.

The means for solving the above problem are as follows.
<1> A Ce-Mo-based composite oxide ceramic containing: cerium; molybdenum; and at least one type of trace element selected from the group consisting of Al, Zr, and Si.
<2> The Ce-Mo-based composite oxide ceramic according to <1> above, wherein an amount of the trace element contained in the Ce-Mo-based composite oxide ceramic is 270 ppm or larger.
<3> The Ce-Mo-based composite oxide ceramic according to <1> or <2> above, wherein the trace element is present in a crystalline phase.
<4> The Ce-Mo-based composite oxide ceramic according to <1> or <2> above, containing Ce₂Mo₃O₁₃.
<5> A green compact made from the Ce-Mo-based composite oxide ceramic according to <1> or <2> above in a powder form, wherein the Ce-Mo-based composite oxide ceramic in the powder form has been compacted.
<6> A sintered body made from the Ce-Mo-based composite oxide ceramic according to <1> or <2> above, wherein the Ce-Mo-based composite oxide ceramic has been sintered.
<7> An article having the Ce-Mo-based composite oxide ceramic according to <1> or <2> above, wherein the Ce-Mo-based composite oxide ceramic is provided to at least a part of a surface of the article.
<8> An article made from the Ce-Mo-based composite oxide ceramic according to <1> or <2> above, wherein the Ce-Mo-based composite oxide ceramic has been dispersed in a base material.

### Advantageous Effect of the Invention

The present invention makes it possible to provide a Ce-Mo-based composite oxide ceramic and other products each having an excellent antibacterial property and an excellent antiviral property.

### BRIEF DESCRIPTION OF THE DRAWINGS

FIG. 1 schematically shows a method for manufacturing a Ce-Mo-based composite oxide ceramic in Example 1 through solid-phase synthesis.
FIG. 2 shows X-ray diffraction spectra regarding respective calcined powders in Example 1 and Comparative Example 1.
FIG. 3 shows changes, over time, in the pHs of pure waters in which the respective calcined powders in Example 1 and Comparative Example 1 were immersed.

### BEST MODE FOR CARRYING OUT THE INVENTION

A Ce-Mo-based composite oxide ceramic of an embodiment contains: cerium (Ce); molybdenum (Mo); and at least one type of trace element selected from the group consisting of Al, Zr, and Si.

The Ce-Mo-based composite oxide ceramic is a composite oxide ceramic containing cerium (Ce) and molybdenum (Mo) and further contains the trace element as an essential component. The composition ratio (molar ratio) of the cerium (Ce) to the molybdenum (Mo) contained in the Ce-Mo-based composite oxide ceramic is not particularly limited unless the object of the present invention is impaired. For example, the composition ratio (molar ratio), i.e., Ce:Mo, may be 2:3 or 1:2.

The amount of oxygen atoms (O) contained in the Ce-Mo-based composite oxide ceramic may be, or does not have to be, based on a stoichiometric composition. That is, the Ce-Mo-based composite oxide ceramic may be a nonstoichiometric compound.

Examples of the Ce-Mo-based composite oxide ceramic include Ce(MoO₄)₂, Ce₂MoO₆, Ce₂(MoO₄)₃, Ce₂Mo₃O₁₃, Ce₂Mo₄O₁₅, Ce₆(MoO₄)₈ (Mo₂O₇), Ce₈Mo₁₂O₄₉, and the like. These types of Ce-Mo-based composite oxide ceramics may be used singly, or two or more of these types of Ce-Mo-based composite oxide ceramics may be used in combination. The Ce-Mo-based composite oxide ceramic is preferably Ce₂Mo₃O₁₃.

The trace element contained in the Ce-Mo-based composite oxide ceramic may be present at any location in the Ce-Mo-based composite oxide ceramic unless the object of the present invention is impaired. However, the trace element is preferably present in a crystalline phase of the Ce-Mo-based composite oxide ceramic from the viewpoint of, for example, making it easy to maintain the Ce-Mo-based composite oxide ceramic to have a preferable crystal structure (e.g., γ-type crystal structure).

As described above, the trace element is at least one type of element selected from the group consisting of Al, Zr, and Si. The trace element is preferably, for example, aluminum.

The amount (proportion) of the trace element contained in the Ce-Mo-based composite oxide ceramic is, for example, preferably 270 ppm or larger, more preferably 500 ppm or larger, and further preferably 700 ppm or larger. The upper limit of the amount (proportion) of the trace element contained in the Ce-Mo-based composite oxide ceramic is not particularly limited unless the object of the present invention is impaired. However, the amount (proportion) is, for example, preferably 100000 ppm or smaller, more preferably 75000 ppm or smaller, and further preferably 50000 ppm or smaller.

It is inferred that, when the amount (proportion) of the trace element contained in the Ce-Mo-based composite oxide ceramic is within this range, the trace element enters a crystal structure of the Ce-Mo-based composite oxide so as to change the crystal structure of the Ce-Mo-based composite oxide so that metal ions forming the crystal structure become easy to elute, whereby the pH at the time of adding the Ce-Mo-based composite oxide ceramic into water decreases. As a result, it is inferred that the antibacterial property and the antiviral property of the Ce-Mo-based composite oxide ceramic become higher than those of a Ce-Mo-based composite oxide ceramic that does not contain the trace element.

The trace element in the Ce-Mo-based composite oxide ceramic is detected by using, for example, an X-ray fluorescence spectrometer (XRF). The amount (proportion) of the trace element contained in the Ce-Mo-based composite oxide ceramic is obtained on the basis of the result of the detection.

The Ce-Mo-based composite oxide ceramic may contain another element (e.g., H, F, Ba, Zn, Mg, Fe, Y, or the like) other than the trace element unless the object of the present invention is impaired. The amount of the other element contained in the Ce-Mo-based composite oxide ceramic may be, for example, 0.01% by mass or lower (100 ppm or smaller) or 0.008% by mass or lower (80 ppm or smaller). The other element in the Ce-Mo-based composite oxide ceramic can be detected by using an X-ray fluorescence spectrometer (XRF) or an ICP optical emission spectrophotometer (ICP).

The Ce-Mo-based composite oxide ceramic may be made from: a crystalline material of a single crystal or a polycrystal; an amorphous material in a glass form or the like; or a combination of the crystalline material portion and the amorphous material portion. The crystal phase of the crystal may be a single phase or a combination of two or more different phases.

A method for manufacturing the Ce-Mo-based composite oxide ceramic is not particularly limited unless the object of the present invention is impaired. Examples of the method include a solid-state reaction method. The method for manufacturing the Ce-Mo-based composite oxide ceramic is preferably a solid-state reaction method from the viewpoint of, for example, making it easy to perform adjustment such that the trace element is contained in the predetermined amount.

The solid-state reaction method as a method for manufacturing the Ce-Mo-based composite oxide ceramic is exemplified below. The solid-state reaction method as a method for manufacturing the Ce-Mo-based composite oxide ceramic includes, for example, a compounding step, a drying step, and a calcination step.

The compounding step is a step of mixing a predetermined amount of a cerium compound, a predetermined amount of a molybdenum compound, and a predetermined amount of the trace element or a trace element compound, to obtain a mixture thereof.

The cerium compound is a compound containing cerium (Ce) necessary for manufacturing the Ce-Mo-based composite oxide ceramic, and examples of the cerium compound include CeO₂, cerium nitrate, cerium chloride, cerium sulfate, cerium hydroxide, cerium carbonate, cerium acetate, and the like. As the cerium compound, at least one type of cerium compound selected from the group consisting of CeO₂, cerium nitrate, cerium chloride, cerium sulfate, cerium hydroxide, cerium carbonate, and cerium acetate may be used. The cerium compound is preferably CeO₂.

The molybdenum compound is a compound containing molybdenum (Mo) necessary for manufacturing the Ce-Mo-based composite oxide ceramic, and examples of the molybdenum compound include MoO₃, MoO₂, MoO, Mo(OH)₃, Mo(OH)₅, and the like. As the molybdenum compound, at least one type of molybdenum compound selected from the group consisting of MoO₃, MoO₂, MoO, Mo(OH)₃, and Mo(OH)₅ may be used. The molybdenum compound is preferably MoO₃.

The mixing ratio of the cerium compound to the molybdenum compound may be adjusted such that the molar ratio of Ce:Mo is 2:3, may be adjusted such that the molar ratio of Ce:Mo is 1:2, may be adjusted such that the molar ratio of Ce:Mo is 1:1, may be adjusted such that the molar ratio of Ce:Mo is 2:1, may be adjusted such that the molar ratio of Ce:Mo is 3:4, or may be adjusted such that the molar ratio of Ce:Mo is 3:5. The mixing ratio of the cerium compound to the molybdenum compound is preferably adjusted such that the molar ratio of Ce:Mo is 2:3.

In the compounding step, the trace element may be used alone, or a trace element compound containing the trace element may be used.

In a case where the trace element is aluminum (Al), examples of the trace element compound (aluminum compound) include Al(NO₃)₃ Al₂O₃, aluminum sulfate, aluminum chloride, and the like. The aluminum compound is preferably Al(NO₃)₃.

In a case where the trace element is zirconium (Zr), examples of the trace element compound (zirconium compound) include zirconium chloride, zirconium hydroxide, zirconium carbonate, zirconium sulfate, zirconium acetate, and the like. The zirconium compound is preferably zirconium chloride.

In a case where the trace element is silicon (Si), examples of the trace element compound (silicon compound) include silicon dioxide, silicon monoxide, silicon carbide, silicon nitride, and the like. The silicon compound is preferably silicon dioxide.

The mixing proportion of the trace element compound to the cerium compound and the molybdenum compound is set as appropriate such that the amount (proportion) of the trace element contained in the Ce-Mo-based composite oxide ceramic falls within the above numerical range.

The compounding step is performed by, for example, putting the predetermined amount of the cerium compound and the predetermined amount of the molybdenum compound into a resin container (resin pot), further putting a predetermined amount of a solvent such as a lower alcohol (ethanol) and the predetermined amount of the trace element compound into the container, and mixing these contents for a predetermined time (e.g., 5 hours to 30 hours) by using a cobble (a cobble made of zirconia, a cobble made of alumina, or the like). Through such a compounding step, a wet mixture in a slurry form is obtained.

The drying step is a step of drying the wet mixture in the slurry form obtained after the compounding step. A method for drying the wet mixture is not particularly limited unless the object of the present invention is impaired. Examples of the method include a drying method with use of a bain-marie, a drying method with use of a vibration dryer, a drying method with use of a spray dryer, and the like.

After the drying step, the wet mixture is dried so as to have a powder form. The obtained mixture in the powder form (mixed powder) is in a state where the cerium compound in a solid phase, the molybdenum compound in a solid phase, and the trace element compound (or the trace element) are mixed with one another.

The calcination step is a step of heating the mixed powder, obtained after the drying step, in a solid phase. For example, in the calcination step, the mixed powder is heated for 1 hour to 24 hours under a condition of a temperature of 400°C or higher and 650°C or lower. The calcination step does not have to be performed in a special synthetic air atmosphere and is performed in an ordinary air atmosphere. The calcination step is not intended for sintering the cerium compound, the molybdenum compound, or the like in the mixed powder.

Through the calcination step, a calcined powder (an example of the Ce-Mo-based composite oxide ceramic) made of a reaction product of the cerium compound, the molybdenum compound, and the trace element compound (or the trace element) is obtained.

The calcined powder may be, as necessary, subjected to granulation through spray drying or the like so as to be prepared in a granular form. Such a powder in a granular form may be used as the Ce-Mo-based composite oxide ceramic.

Alternatively, a sintered body obtained by firing and sintering the calcined powder may be used as the Ce-Mo-based composite oxide ceramic. For example, the calcined powder is compacted into a predetermined shape (e.g., a columnar shape, a disc shape, or the like) by using a predetermined press machine, and the obtained compacted product (green compact) is heated for 1 hour to 24 hours under a predetermined temperature condition (e.g., 400°C or higher and 650°C or lower) so as to be sintered, whereby a sintered body usable as the Ce-Mo-based composite oxide ceramic is obtained.

A firing step is a heating step for sintering the Ce-Mo-based composite oxide ceramic in the calcined powder and can be performed in an air atmosphere.

Alternatively, the Ce-Mo-based composite oxide ceramic of the present embodiment may be manufactured as appropriate by applying, for example, citric acid polymerization or hydrothermal synthesis described in International Publication WO 2022/014631.

The Ce-Mo-based composite oxide ceramic of the present embodiment exhibits an antibacterial property (antibacterial activity) and an antiviral property (antiviral activity) in a pre-sintered state. In the same manner as before being sintered, the Ce-Mo-based composite oxide ceramic of the present embodiment exhibits the antibacterial property and the antiviral property also after being sintered. Such a Ce-Mo-based composite oxide ceramic can be used as an antibacterial agent having the antibacterial property, an antiviral agent having the antiviral property, or an antibacterial and antiviral agent having both the antibacterial property and the antiviral property.

The antibacterial property and the antiviral property of the Ce-Mo-based composite oxide ceramic can be evaluated according to, for example, a film cover method that conforms to JIS R 1756. For example, the virus reduction rate of the Ce-Mo-based composite oxide ceramic measured according to the film cover method after 24 hours elapse, is 99% or higher.

The form of the Ce-Mo-based composite oxide ceramic of the present embodiment is not particularly limited and may be a desired form according to use unless the object of the present invention is impaired. For example, the Ce-Mo-based composite oxide ceramic may be in a powder form or in a granular form obtained by granulating the powder through spray drying or the like. Alternatively, the Ce-Mo-based composite oxide ceramic may be used in the form of a green compact obtained by compacting the ceramic material in the powder form. Alternatively, the Ce-Mo-based composite oxide ceramic may be used in the form of a sintered body.

Alternatively, the Ce-Mo-based composite oxide ceramic may be used in a state of being applied to at least a part of a surface of an article. A material for forming the article to which the Ce-Mo-based composite oxide ceramic is to be applied is not particularly limited unless the object of the present invention is impaired. Examples of the material include glass, ceramic, synthetic resins such as thermoplastic resin and thermosetting resin, rubbers (natural rubber and synthetic rubber), genuine leather (natural leather), synthetic leather, metal-based materials each made from a metal or an alloy, wood, paper, fiber, nonwoven fabric, silicon (silicon wafers and the like), carbon materials, minerals, gypsum, and the like.

Alternatively, the Ce-Mo-based composite oxide ceramic may be used in a state of being dispersed in a predetermined base material. The base material in which the Ce-Mo-based composite oxide ceramic is to be dispersed is not particularly limited unless the object of the present invention is impaired. Examples of the base material include glass, ceramic, synthetic resins such as thermoplastic resin and thermosetting resin, rubbers (natural rubber and synthetic rubber), synthetic leather, metal-based materials each made from a metal or an alloy, paper, fiber, nonwoven fabric, carbon materials, minerals, gypsum, and the like.

Hereinafter, the present invention will be further described on the basis of Examples. However, the present invention is not limited in any way by the Examples.

### [Example 1]

CeO₂ was prepared as a cerium compound, MoO₃ was prepared as a molybdenum compound, and Al(NO₃)₃ was prepared as a trace element compound (aluminum compound). Then, a raw material powder of the cerium compound and a raw material powder of the molybdenum compound were weighed out such that the molar ratio therebetween was 2:3 (Ce:Mo=2:3). Each of the raw material powders having been weighed out was put into a resin container (resin pot), a predetermined amount of ethanol and a predetermined amount of the trace element compound (Al(NO₃)₃) were further put into the container, and these contents were mixed for 24 hours by using a cobble made of zirconia (compounding step). The obtained wet mixture in a slurry form was dried with a bain-marie, whereby a mixed powder was obtained (drying step). The mixed powder was in a state where the cerium compound in a solid phase, the molybdenum compound in a solid phase, and the trace element compound were mixed with one another.

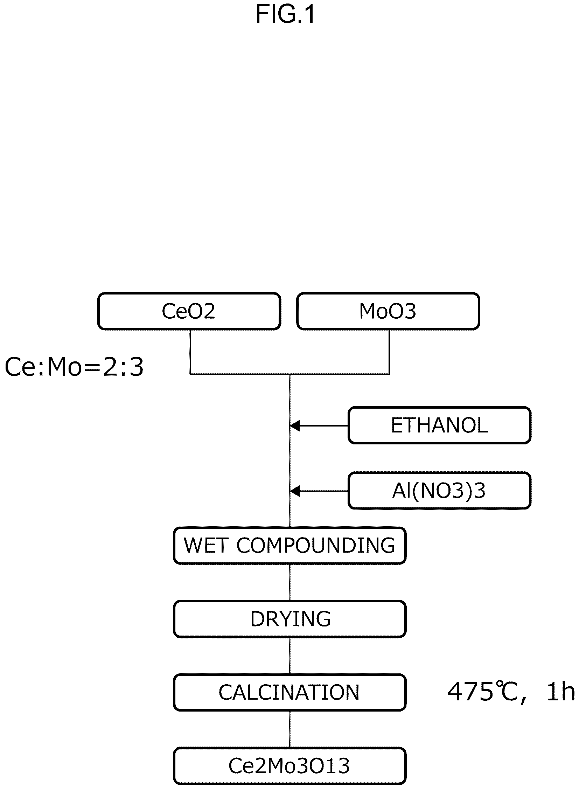

Subsequently, the mixed powder was calcined for 1 hour under a condition of a temperature of 475°C in an air atmosphere, whereby a calcined powder (Ce-Mo-based composite oxide ceramic) made of a reaction product of the cerium compound, the molybdenum compound, and the trace element compound was obtained. FIG. 1 schematically shows the method for manufacturing the Ce-Mo-based composite oxide ceramic in Example 1 through the solid-phase synthesis.

### [Comparative Example 1]

Manufacturing was performed in the same manner as in Example 1, except that the trace element compound (aluminum compound) was not mixed with the raw material powder of the cerium compound and the raw material powder of the molybdenum compound. Consequently, a calcined powder made of a reaction product of the cerium compound and the molybdenum compound was obtained.

### [Identification of Crystalline Phase through XRD]

Identification of a crystalline phase was performed on each of the calcined powders in Example 1 and Comparative Example 1 through powder X-ray diffraction (XRD). Measurement conditions are as follows.

### <Measurement Conditions>

Measurement device: a powder X-ray diffraction device (device name "Smart lab" manufactured by Rigaku Corporation)
Detector: D/teX Ultra 250
Optical system: a concentrated optical system of a Bragg-Brentano type
X-ray output: 40 kV-30 mA
Step width: 0.0100°
Scan axis: 2θ/θ
Scan range: 10.00° to 80.00°

### <Measurement Results>

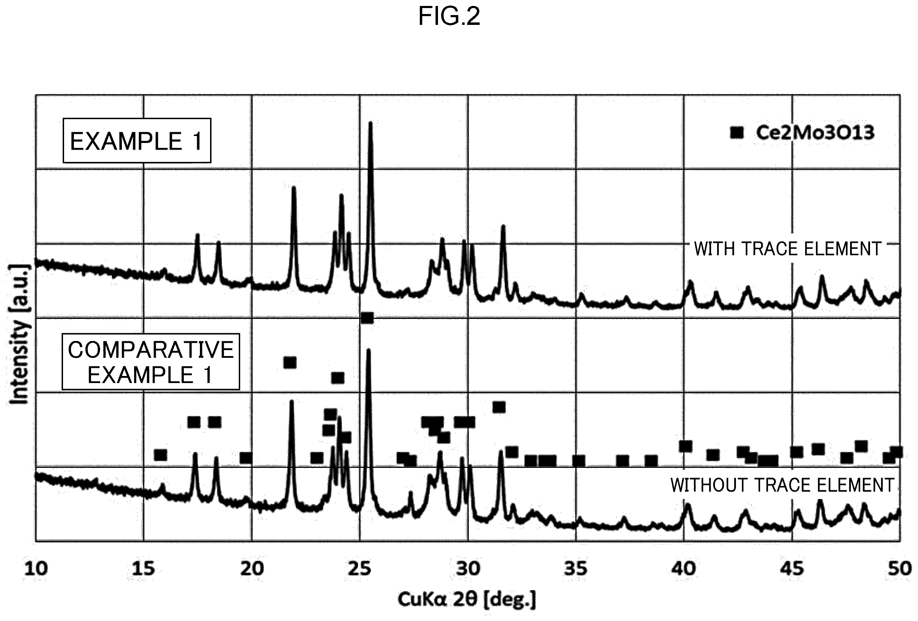

The measurement results of the XRD (X-ray diffraction spectra) are shown in FIG. 2. FIG. 2 shows the X-ray diffraction spectra regarding the respective calcined powders in Example 1 and Comparative Example 1. The measurement result regarding Example 1 is shown on the upper side of FIG. 2, and the measurement result regarding Comparative Example 1 is shown on the lower side of FIG. 2. Judging from X-ray diffraction peaks of the measurement result shown on the lower side of FIG. 2, it has been confirmed that the calcined powder in Comparative Example 1 had a crystal structure of Ce₂Mo₃O₁₃.

Also, judging from X-ray diffraction peaks of the measurement result shown on the upper side of FIG. 2, it has been confirmed that the calcined powder in Example 1 had a crystal structure of Ce₂Mo₃O₁₃ similarly to Comparative Example 1. It is inferred that not only Ce₂Mo₃O₁₃ but also aluminum (Al) as the trace element was contained in the crystalline phase of the calcined powder in Example 1. That is, it is inferred that the elements of the Ce₂Mo₃O₁₃ (Ce-Mo-based composite oxide ceramic) forming the crystalline phase in Example 1 were partially substituted with the aluminum (trace element).

### [Change in pH at Time of Immersion in Pure Water]

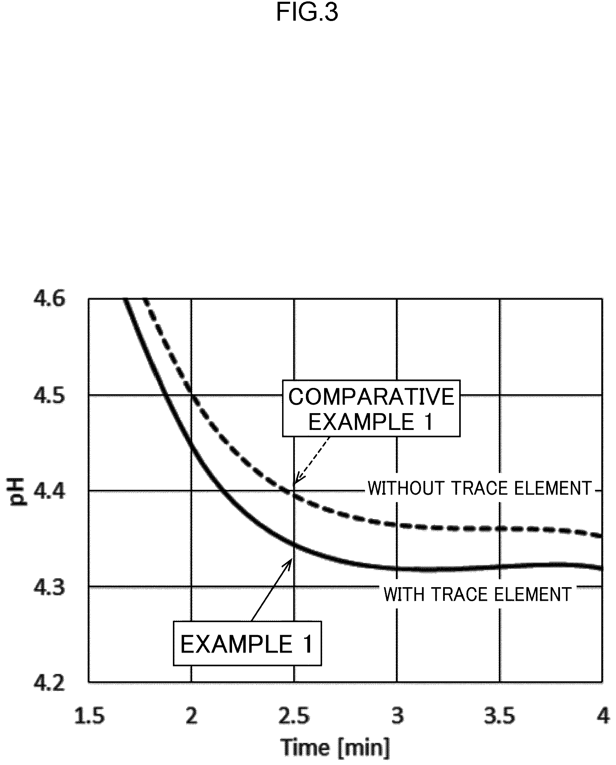

The calcined powder (100 mg) in Example 1 was immersed in pure water (100 ml) inside a container made of glass, and the pure water in which the calcined powder was immersed was stirred and mixed. Then, change, over time, in the pH of the pure water in which the calcined powder was immersed was measured by using a pH meter with the time of the immersion of the calcined powder being regarded as 0 hours. In addition, regarding the calcined powder in Comparative Example 1 as well, change, over time, in the pH of pure water in which the calcined powder was immersed was measured in the same manner. The results are shown in FIG. 3.

FIG. 3 shows (as graphs) the changes, over time, in the pHs of the pure waters in which the respective calcined powders in Example 1 and Comparative Example 1 were immersed. In FIG. 3, the graph indicated by the solid line corresponds to Example 1, and the graph indicated by the broken line corresponds to Comparative Example 1. As shown in FIG. 3, it has been confirmed that, in Example 1, the pH value is smaller, i.e., the acidity is higher, than in Comparative Example 1 at any time. This is inferred to be because, owing to influence of the aluminum (trace element) contained in the calcined powder in Example 1, a change occurred in the crystal structure of the Ce-Mo-based composite oxide ceramic (Ce₂Mo₃O₁₃) so that metal ions became easy to elute from the calcined powder, whereby the pH of the pure water in which the calcined powder was immersed decreased.

## Claims

1. A Ce-Mo-based composite oxide ceramic comprising:
cerium;
molybdenum; and
at least one type of trace element selected from the group consisting of Al, Zr, and Si.

2. The Ce-Mo-based composite oxide ceramic according to claim 1, wherein an amount of the trace element contained in the Ce-Mo-based composite oxide ceramic is 270 ppm or larger.

3. The Ce-Mo-based composite oxide ceramic according to claim 1 or 2, wherein the trace element is present in a crystalline phase.

4. The Ce-Mo-based composite oxide ceramic according to claim 1 or 2, comprising Ce₂Mo₃O₁₃.

5. A green compact made from the Ce-Mo-based composite oxide ceramic according to claim 1 or 2 in a powder form, wherein the Ce-Mo-based composite oxide ceramic in the powder form has been compacted.

6. A sintered body made from the Ce-Mo-based composite oxide ceramic according to claim 1 or 2, wherein the Ce-Mo-based composite oxide ceramic has been sintered.

7. An article comprising the Ce-Mo-based composite oxide ceramic according to claim 1 or 2, wherein the Ce-Mo-based composite oxide ceramic is provided to at least a part of a surface of the article.

8. An article made from the Ce-Mo-based composite oxide ceramic according to claim 1 or 2, wherein the Ce-Mo-based composite oxide ceramic has been dispersed in a base material.
